(19) Europäisches Patentamt
European Patent Office
Office européen des brevets

(11) **EP 2 351 524 B1**

(12) **EUROPEAN PATENT SPECIFICATION**

(45) Date of publication and mention
of the grant of the patent:
**06.05.2020   Bulletin 2020/19**

(51) Int Cl.:
*A61B 5/18* *(2006.01)*          *B60K 28/06* *(2006.01)*
*A61B 5/11* *(2006.01)*          *A61B 5/00* *(2006.01)*

(21) Application number: **09814570.9**

(22) Date of filing: **15.09.2009**

(86) International application number:
**PCT/JP2009/066093**

(87) International publication number:
**WO 2010/032725 (25.03.2010 Gazette 2010/12)**

(54) **DROWSINESS DETERMINING DEVICE AND PROGRAM**

VORRICHTUNG UND PROGRAMM ZUR BESTIMMUNG VON SCHLÄFRIGKEIT

PROGRAMME ET DISPOSITIF PERMETTANT DE DÉTERMINER L'ÉTAT DE SOMNOLENCE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **16.09.2008   JP 2008236432**

(43) Date of publication of application:
**03.08.2011   Bulletin 2011/31**

(73) Proprietor: **Denso Corporation
Kariya-city,
Aichi 448-8661 (JP)**

(72) Inventors:
• **YODA, Takumi**
  **Aichi-gun**
  **Aichi 480-1192 (JP)**
• **TERASHIMA, Ryuta**
  **Aichi-gun**
  **Aichi 480-1192 (JP)**
• **TSUDA, Taishi**
  **Toyota-shi**
  **Aichi 471-8571 (JP)**
• **OMI, Takuhiro**
  **Kariya-shi**
  **Aichi 448-8661 (JP)**

(74) Representative: **TBK
Bavariaring 4-6
80336 München (DE)**

(56) References cited:
JP-A- 9 147 120          JP-A- 2004 041 485
JP-A- 2004 041 485       JP-A- 2007 151 798
JP-B2- 3 286 887         US-A- 5 786 765

**Description**

Technical Field

The present invention relates to a drowsiness determination apparatus and program, and in particular to a drowsiness determination apparatus and program for determining the state of drowsiness of a vehicle driver.

Background Art

A open-eye or closed-eye monitoring apparatus is already known (see Japanese Patent Application Laid-Open (JP-A) No. 2004-41485) in which a degree of eye openness from image capture means is detected, plural minimum values of degree of eye openness are extracted from the openness in a specific duration of time-varying data, the plural minimum values are separated into an open-eye candidate group and a closed-eye candidate group, and openness of a value of the minimum openness among the open-eye candidates minus the standard deviation of the open-eye candidate group or less, or openness of a value of the minimum openness among the open-eye candidates plus the standard deviation of the closed-eye candidate group or greater is set as a closed eye threshold value. An accurate value of closed eye threshold value can be set according to such an open-eye or closed-eye monitoring apparatus.

Prior art can be found e.g. in document JP H03-286887B2, also published as JP3286887B2, disclosing a face imaging processing device, in document JP 2004 041485 A disclosing a closed/open eye monitoring device, in document US 5,786,765 A disclosing an apparatus for estimating the drowsiness level of a vehicle driver.

DISCLOSURE OF THE INVENTION

Technical Problem

However, there is an issue in the technology of JP-A No. 2004-41485 in that depending on the type of blinking feature amount desired for extraction, cases arise where the appropriate threshold value for extracting a blinking feature amount is different from the accurate threshold value for setting. For example, when a sustained duration of closed-eye is desired for extraction as the blinking feature amount, unless a higher degree of eye openness is set than the conventional closed eye threshold value, when the detected openness moves up down in the vicinity of the closed eye threshold value due to noise or the like, this sometimes leads to the blinking feature amount being incorrectly extracted.

The present invention is made to address the above issue, and an object thereof is to provide a drowsiness determination apparatus and program that are capable of extracting blinking feature amounts in which appropriate respective threshold values are employed for the type of blinking feature amount, and determining the state of drowsiness with good precision.

Solution to Problem

In order to achieve the above objective, a drowsiness determination apparatus according to the present invention is configured including: an image capture means for capturing a region including an eye of a determination subject; an openness detection means for detecting a degree of eye openness based on the image captured by the image capture means; a feature amount extraction means for, based on the degree of eye openness detected by the openness detection means, extracting plural types of blinking feature amount selected from the group consisting of a blinking feature amount related to grouping of blinking, derived by employing a threshold value for degree of eye openness smaller than a standard threshold value, a blinking feature amount related to a number of blinks, derived by employing the standard threshold value and differing from the blinking feature amount related to the grouping of blinking, a blinking feature amount relating to length of open-eye state in a given time period, derived by employing a threshold value greater than the standard threshold value and a value corresponding to the entire length of open-eye state in the given time period, a blinking feature amount relating to length of open-eye state in a given time period, derived by employing a threshold value smaller than the standard threshold value and a value corresponding to the length of a portion of open-eye state in the given time period, a blinking feature amount relating to length of closed-eye state in a given time period, derived by employing a threshold value smaller than the standard threshold value and a value corresponding to the entire length of closed-eye state in the given time period, and a blinking feature amount relating to length of closed-eye state in a given time period, derived by employing a threshold value greater than the standard threshold value and a value corresponding to the length of a portion of the closed-eye state in the given time period; and a state of drowsiness determination means for determining the state of drowsiness of the determination subject based on the plural types of blinking feature amount extracted by the feature amount extraction means.

A program according to the present invention is a program for causing a computer to function as: an openness detection means for detecting a degree of eye openness based on an image captured by an image capture means for capturing a region including an eye of a determination subject; a feature amount extraction means for, based on the degree of eye openness detected by the openness detection means, extracting plural types of blinking feature amount selected from the group consisting of a blinking feature amount related to grouping of blinking, derived by employing a threshold value for degree of eye openness smaller than a standard threshold value, a blinking feature amount related to a number of blinks, derived by employing the standard threshold value and differing from the blinking feature amount related to the grouping of blinking, a blinking feature amount relating to length of open-eye state in a given time period, derived by employing a threshold value greater than the standard threshold value and a value corresponding to the entire length of open-eye state in the given time period, a blinking feature amount relating to length of open-eye state in a given time period, derived by employing a threshold value smaller than the standard threshold value and a value corresponding to the length of a portion of open-eye state in the given time period, a blinking feature amount relating to length of closed-eye state in a given time period, derived by employing a threshold value smaller than the standard threshold value and a value corresponding to the entire length of closed-eye state in the given time period, and a blinking feature amount relating to length of closed-eye state in a given time period, derived by employing a threshold value greater than the standard threshold value and a value corresponding to the length of a portion of the closed-eye state in the given time period; and a state of drowsiness determination means for determining the state of drowsiness of the determination subject based on the plural types of blinking feature amount extracted by the feature amount extraction means.

According to the present invention, an image is captured of a region including the eye of the determination subject with the image capture means, and the degree of eye openness is detected by the openness detection means based on the image captured by the image capture means.

Then, using the feature amount extraction means and based on the degree of eye openness detected by the openness detection means, plural types of blinking feature amount are extracted, as selected from the group consisting of the blinking feature amount related to grouping of blinking, derived by employing the threshold value for degree of eye openness smaller than a standard threshold value, the blinking feature amount related to a number of blinks, derived by employing the standard threshold value and differing from the blinking feature amount related to the grouping of blinking, a blinking feature amount relating to length of open-eye state in a given time period, derived by employing a threshold value greater than the standard threshold value and a value corresponding to the entire length of open-eye state in the given time period, a blinking feature amount relating to length of open-eye state in a given time period, derived by employing a threshold value smaller than the standard threshold value and a value corresponding to the length of a portion of open-eye state in the given time period, a blinking feature amount relating to length of closed-eye state in a given time period, derived by employing a threshold value smaller than the standard threshold value and a value corresponding to the entire length of closed-eye state in the given time period, and a blinking feature amount relating to length of closed-eye state in a given time period, derived by employing a threshold value greater than the standard threshold value and a value corresponding to the length of a portion of the closed-eye state in the given time period.

Then, using the state of drowsiness determination means, the state of drowsiness of the determination subject is determined based on the plural types of blinking feature amount extracted by the feature amount extraction means.

Accordingly, by employing the standard threshold value, the smaller threshold value, or the greater threshold value for the degree of eye openness to extract the plural blinking feature amounts, the blinking feature amounts can be extracted using an appropriate respective threshold value for the plural blinking feature amounts, and hence the state of drowsiness can be determined with good precision.

The drowsiness determination apparatus according to the present invention can be configured to further include a threshold value computation means for computing the standard threshold value, the threshold value greater than the standard threshold value and the threshold value smaller than the standard threshold value the threshold value computation means for, based on the degree of eye openness detected by the openness detection means.

The above threshold value computation means can compute the standard threshold value, the threshold value greater than the standard threshold value and the threshold value smaller than the standard threshold value, based on the distribution of degree of eye openness obtained from the degree of eye openness detected by the openness detection means. The above threshold value computation means can extract a maximum value with larger degree of eye openness and a maximum value with smaller degree of eye openness from two maximum values in the degree of eye openness distribution, together with a minimum value present between the two maximum values, and then compute the standard threshold value based on the minimum value, compute the threshold value smaller than the standard threshold value based on the minimum value and the maximum value of greater degree of eye openness, and compute the threshold value smaller than the standard threshold value based on the minimum value and the maximum value of larger degree of eye openness.

Advantageous Effects of the Invention

As explained above, according to the drowsiness determination apparatus and the program of the present invention, by extracting plural types of blinking feature amount by employing the standard threshold value for degree of eye openness, the smaller threshold value or the greater threshold value, blinking feature amounts can be extracted by employing an appropriate respective threshold value for the plural types of blinking feature amount, thereby obtaining the effect of determining the state of drowsiness with good precision.

BRIEF DESCRIPTION OF THE DRAWINGS

Fig. 1 is a schematic diagram showing a configuration of a drowsiness determination apparatus according to a first exemplary embodiment of the present invention.
Fig. 2 is a graph showing a waveform of degree of eye openness and closed eye threshold values.
Fig. 3 is a graph showing a frequency distribution of degree of eye openness and time series data of degree of eye openness.
Fig. 4 is a graph showing a frequency distribution of degree of eye openness.
Fig. 5 is a flow chart showing contents of a threshold value calculation processing routine in a computer of a drowsiness determination apparatus according to the first exemplary embodiment of the present invention.
Fig. 6 is a flow chart showing contents of a drowsiness determination processing routine in a computer of a drowsiness determination apparatus according to the first exemplary embodiment of the present invention.

BEST MODE FOR CARRYING OUT THE INVENTION

Detailed explanation follows regarding exemplary embodiments of the present invention, with reference to the drawings. Explanation is given of examples of application of the present invention to a vehicle-mounted drowsiness determination apparatus.

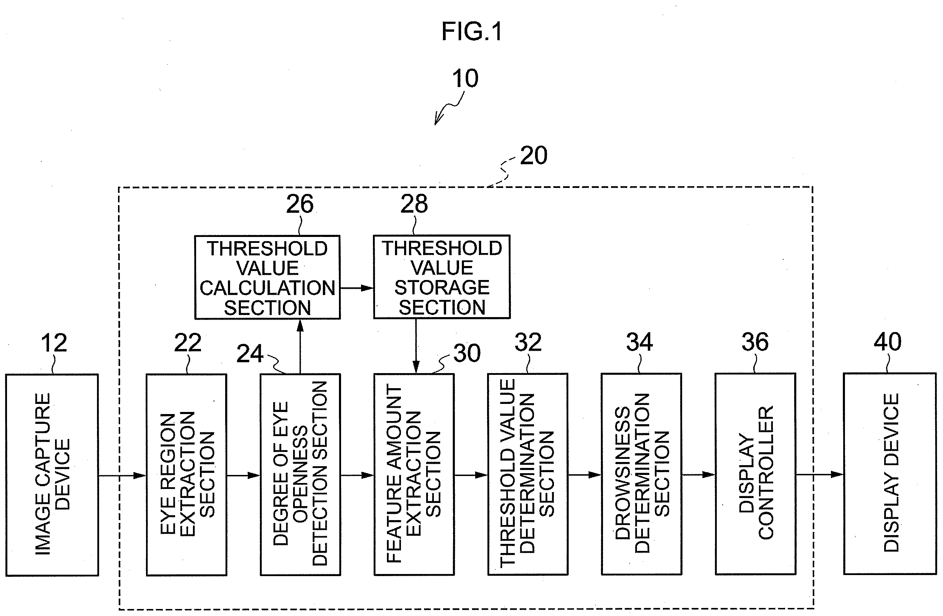

A drowsiness determination apparatus 10 according to a first exemplary embodiment, as shown in Fig. 1, is equipped with an image capture device 12, for example, mounted diagonally in front of a driver, serving as a determination subject, for capturing an image of the face of the driver from diagonally above, and a computer 20 for performing state of drowsiness determination based on the face image captured by the image capture device 12, and displaying the determination result on a display device 40.

The computer 20 is equipped with a CPU, RAM, and ROM stored with a program for executing a threshold value calculation processing routine and a drowsiness determination processing routine, described later. The computer 20 is functionally configured as set out below. The computer 20 includes: an eye region extraction section 22 for extracting from a face image an eye region representing an eye of a driver; a degree of eye openness detection section 24 for detecting the degree of eye openness expressing the extent an eye has been opened; a threshold value calculation section 26 for calculating plural types of closed eye threshold value with differing magnitudes from detected degree of eye openness time series data; a threshold value storage section 28 for storing the plural types of calculated closed eye threshold value; a feature amount extraction section 30 for extracting plural types of blinking feature amounts from detected degree of eye openness time series data by employing the stored plural types of closed eye threshold value; a threshold value determination section 32 that performs threshold value determination on each of the extracted blinking feature amounts; a drowsiness determination section 34 for determining a state of drowsiness based on the results of threshold value determination for each of the respective plural types of blinking feature amount; and a display controller 36 that displays warning information on the display device 40 when a nodding-off state is determined to exist.

The degree of eye openness detection section 24 detects the degree of eye openness based on a ratio of the distance between upper eyelid and lower eyelid detected in the image of the eye region with respect to a predetermined distance between the upper eyelid and the lower eyelid when fully open. The degree of eye openness detection section 24 detects the degree of eye openness, with 100% being when the eye is fully open, and 0% when fully closed. Degree of eye openness is detected as being, for example, 50%, when drowsiness occurs with the eye half open. Note that configuration may be made such that the distance between upper eyelid and lower eyelid is detected as the degree of eye openness.

Explanation now follows regarding the principles of the present exemplary embodiment. Degree of eye openness obtained by image analysis or the like has incorporated noise components caused by movement of the image capture subject, wrong detection temporality, lost tracking temporality and the like. This noise is sometimes interpreted as occurrences of blinking, with noise components causing failures in correct extraction from the degree of eye openness of blinking feature amounts employed in state of drowsiness determination.

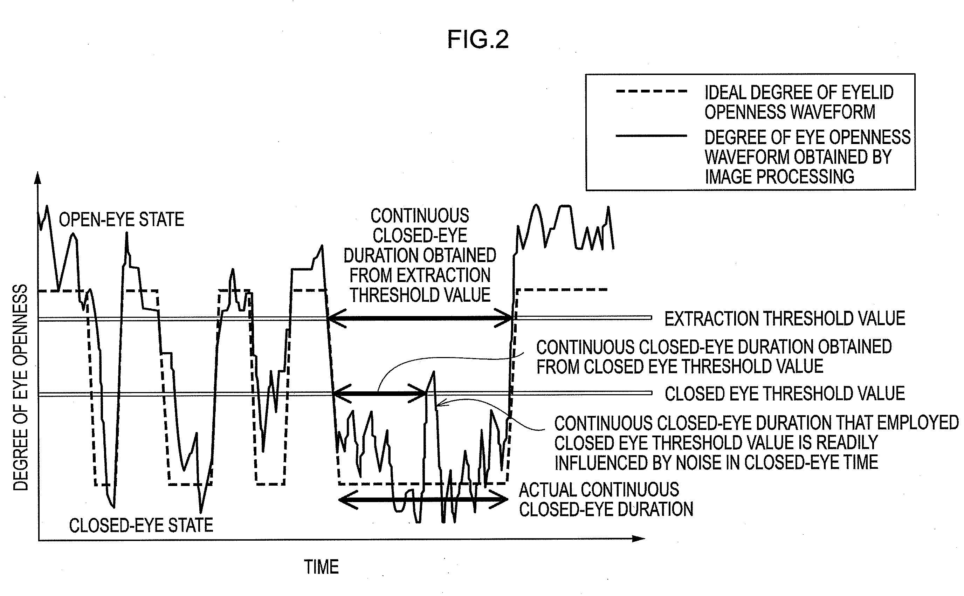

For example, in a conventional blinking feature amount extraction method, since a closed-eye state and an open-eye state are determined employing a constant closed eye threshold value when plural types of blinking feature amount are extracted, there is sometimes is a large influence from noise during the closed-eye state on a continuous open-eye duration extracted using the closed eye threshold value, as shown in Fig. 2. However, by setting an extraction threshold value for the blinking feature amount desired for extraction (for example, a distribution of continuous closed-eye durations) such that the influence from noise is minimized (for example, a threshold value greater than the normal closed eye threshold value), blinking feature amounts are extracted close to blinking feature amounts obtained from an ideal waveform of degree of eye openness, resulting in determination of state of drowsiness being performable with good precision.

Hence, in the present exemplary embodiment, as explained below, the threshold value calculation section 26 respectively calculates, as extraction threshold values, a standard closed eye threshold value, a closed eye threshold value with a value larger than the standard closed eye threshold value (a closed eye threshold value near to the open-eye state), and a closed eye threshold value with a value smaller than the standard closed eye threshold value (a closed eye threshold value near to the closed-eye state).

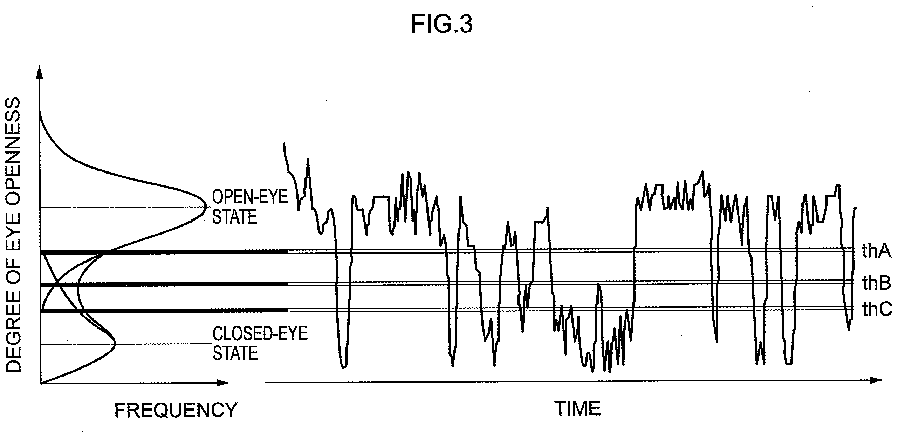

First, as shown in Fig. 3, a frequency distribution of degree of eye openness is generated from a specific duration's worth of detected degree of eye openness time series data. This is shown on a graph in above Fig. 3, using a common vertical axis to the degree of eye openness. As can be seen from the degree of eye openness time series data shown in Fig. 3, there is noise changes occurring in the degree of eye openness waveform of smaller amplitude than changes due to blinking, even when an open-eye state or a closed-eye state continues. Plural types of extraction threshold value are calculated with different magnitudes, in order to reduce by as much as possible the influence of such noise on blinking feature amounts.

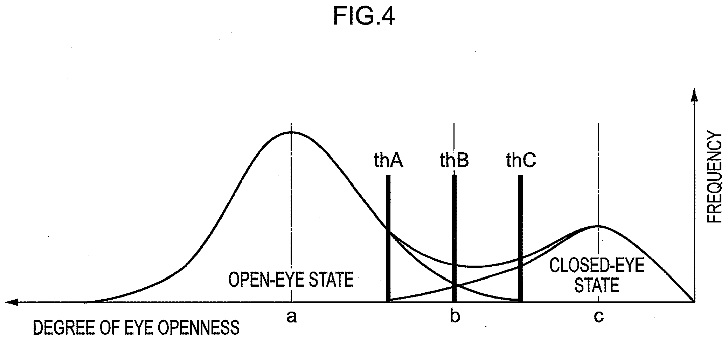

As shown in Fig. 3, the degree of eye openness frequency is split into open-eye states and closed-eye states, by analyzing the frequency distribution of Fig. 3. As shown in Fig. 4, two maxima values of the frequency distribution consist of a maximum value open-eye state histogram peak a at large degree of eye openness and a maximum value closed-eye state histogram peak c at small degree of eye openness, with a minimum value b present between these peaks.

A large value closed eye threshold value thA, a standard closed eye threshold value thB, and a small value closed eye threshold value thC are calculated according to Equation (1) to Equation (3) below,

$$thA = b + (b - c) \times Xa \qquad \text{Equation (1)}$$

$$thB = b \qquad \text{Equation (2)}$$

$$thC = b + (a - b) \times Xb \qquad \text{Equation (3)}$$

wherein Xa and Xb are specific constants.

Note that configuration may be made such that the large value closed eye threshold value thA, the standard closed eye threshold value thB and the small value closed eye threshold value thC are calculated according to Equation (4) to Equation (6) below,

$$thA = b + Xa \qquad \text{Equation (4)}$$

$$thB = b \qquad \text{Equation (5)}$$

$$thC = b - Xb \qquad \text{Equation (6)}$$

wherein Xa and Xb are specific constants.

Note that the large value closed eye threshold value thA calculated in above Equation (1) and Equation (4) is a threshold value for a maximum limit extraction range of the closed-eye state so as to limit the influence from noise in the closed-eye state, and is a threshold value for ensuring that an open-eye state is not incorrectly extracted due to noise during a closed-eye state. Furthermore, the small value closed eye threshold value thC calculated in above Equation (3) and Equation (6) is a threshold value for a maximum limit extraction range of the open-eye state so as to limit influence from noise in the open-eye state, and is a threshold value for ensuring that a closed-eye state is not incorrectly extracted due to noise during an open-eye state.

The feature amount extraction section 30 extracts plural types of blinking feature amount using the plural types of closed eye threshold value, as explained below.

First, when extracting blinking feature amount relating to the length of a closed-eye state in the given time period by derivation using a value corresponding to the length of a portion of a closed-eye state during a given time period, the feature amount extraction section 30 employs the large value closed eye threshold value thA as the extraction threshold value so as to limit the influence from noise in the closed-eye state, to extract a maximum range of the closed-eye state and to derive a value corresponding to the length of a portion of closed-eye state during the given time period with good precision. Accordingly, the closed-eye state is extracted from the degree of eye openness time series data less than the large value closed eye threshold value thA, and a value corresponding to the length of a portion of closed-eye state in the given time period is derived with good precision, and the blinking feature amount extracted.

A continuous closed-eye duration distribution is, for example, derived from values using a sustained duration of closed-eye state from the closed-eye state inside a specific extraction time frame. As shown in Fig. 3, were the standard closed eye threshold value thB or the small value closed eye threshold value thC to be employed as the extraction threshold value, then even during a sustained closed-eye state the extraction threshold value would sometimes be exceeded due to the influence of noise, and extraction of continuous closed-eye durations could not be performed with good precision. However, by employing the large value closed eye threshold value thA, as shown in Fig. 3, in order to extract a continuous closed-eye duration distribution as the blinking feature amount, the feature amount extraction section 30 measures the continuous duration of closed-eye state with good precision using the maximum limit extraction range of closed-eye state by extracting from the degree of eye openness time series data closed-eye states of less than the large value closed eye threshold value thA, thereby extracting the continuous closed-eye duration distribution.

Further, in order to extract a blinking feature amount related to number of blinks, the feature amount extraction section 30 employs the standard closed eye threshold value thB as the extraction threshold value, so as to extract blinks that are changes in one direction or the other between the open-eye state and the closed-eye state with small influence from noise in both the open-eye state and the closed-eye state. The feature amount extraction section 30 accordingly extracts the number of blinks with good precision by using the standard closed eye threshold value thB to extract the number of times the standard closed eye threshold value thB is exceeded in the degree of eye openness time series data, thereby extracting the blinking feature amount.

For example, when the number of blinks is taken as the number of times of to-and-fro above and below a closed eye threshold value occurring during a specific extraction time frame, the feature amount extraction section 30 employs the standard closed eye threshold value thB to extract changes in degree of eye openness exceeding the standard closed eye threshold value thB from the degree of eye openness time series data, measuring the number of times to-and-fro above and below the closed eye threshold value, and thereby extracting the number of blinks.

When extracting a blinking feature amount related to the length of closed-eye state during a given time period by derivation using a value corresponding to the entire length of closed-eye state in the given time period, noise in the closed-eye state does not readily have an influence. However, were the closed eye threshold value thA near to the open-eye state to be employed, then the closed eye threshold value thA would be sometimes be exceeded even when the eye has not actually been fully closed, resulting in incorrect determination as a closed-eye state. Accordingly, in order that the closed-eye state can be extracted more correctly, the feature amount extraction section 30 extracts less than the closed eye threshold value thC as a closed-eye state from the degree of eye openness time series data by employing the small value closed eye threshold value thC as the extraction threshold value. Therefore, the feature amount extraction section 30 extracts the closed-eye state with good precision, and thereby extracts a value corresponding to the entire length (duration) of closed-eye state in the given time period as the blinking feature amount.

When the proportion of closed-eye is, for example, taken as the proportion of closed-eye state in a specific extraction time frame, and since it is dependent on the integral value of durations of closed-eye state in the extraction time frame, noise in the closed-eye state does not readily have an influence. Hence, in order to extract the proportion of closed-eye as the blinking feature amount, the feature amount extraction section 30 extracts less than the closed eye threshold value thC as the closed-eye state from the degree of eye openness time series data by employing the small value closed eye threshold value thC. Therefore, the closed-eye state is extracted with good precision such that the closed-eye state is not incorrectly extracted due to noise in the open-eye state,, an integral value of the duration of closed-eye state is computed, and the proportion of closed-eye thereby extracted.

In the present exemplary embodiment, explanation is given of examples of extracting the continuous closed-eye duration distribution, the number of blinks, and the proportion of closed-eye as the plural types of blinking feature amount.

The threshold value determination section 32 performs threshold value determination for each of the extracted plural types of blinking feature amount, and determines whether or not the blinking feature amount corresponds to a nodding-off state. For example, determination is made as to whether or not the extracted continuous closed-eye duration is a threshold value $th_{Dur}$ relating to the continuous closed-eye duration or greater, and determination is made as to whether or not the extracted number of blinks is a threshold value $th_{CNT}$ relating to the number of blinks or greater. Furthermore, determination is made as to whether or not the extracted proportion of closed-eye is a threshold value $th_{CLS}$ related to proportion of closed-eye or greater.

The drowsiness determination section 34 determines that the determination subject, the driver, is in a nodding-off state when determined by threshold value determination on each of the plural types of extracted blinking feature amount that all types of the blinking feature amount are blinking feature amounts corresponding to a nodding-off state.

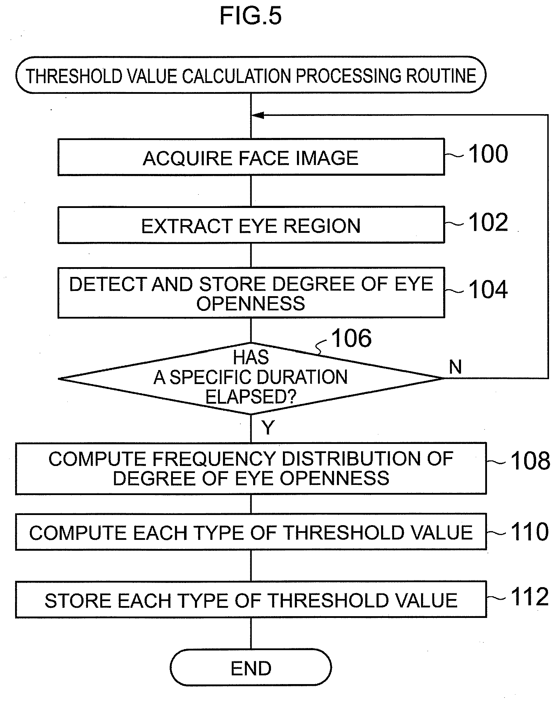

Explanation now follows regarding operation of the drowsiness determination apparatus 10 according to the first exemplary embodiment. First, images are successively captured of the face of the driver by the image capture device 12, and the threshold value calculation processing routine shown in Fig. 5 is executed in the computer 20.

The face images are acquired from the image capture device 12 at step 100, and the eye region is extracted from the acquired face image at step 102.

Then, at step 104, the degree of eye openness is computed based on the image of the extracted eye region and stored in a memory (not shown in the drawings). Then, at step 106, determination is made as to whether or not a specific duration has elapsed since starting processing. Processing returns to step 100 when the specific duration has not yet elapsed, and processing proceeds to step 108 when the specific duration has elapsed.

A time series of degree of eye openness detected during the specific duration is thereby stored in the memory by step 100 to step 106.

At step 108, the degree of eye openness frequency distribution is computed from the degree of eye openness time series data stored in the memory. At step 110, the maximum values and the minimum value obtained in the degree of eye openness frequency distribution computed at step 108 are employed, and the standard closed eye threshold value, large value closed eye threshold value, and the small value closed eye threshold value are each computed according to Equation (1) to Equation (3).

Then, at step 112, the plural types of closed eye threshold value computed at step 110 are stored in the threshold value storage section 28, thereby completing the threshold value calculation processing routine.

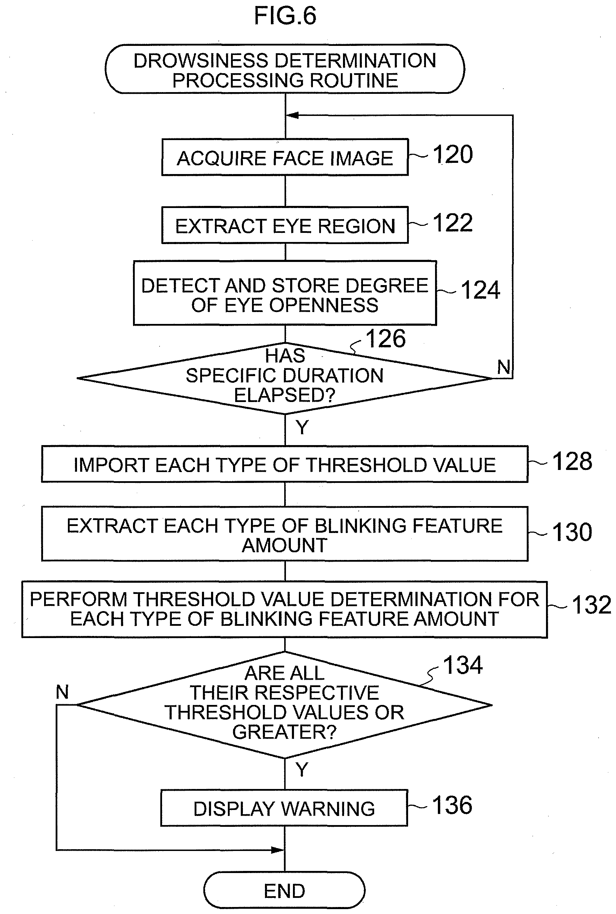

Once the plural types of closed eye threshold value have been calculated by the above threshold value calculation processing routine, images are then successively captured of the face of the driver by the image capture device 12, and the drowsiness determination processing routine shown in Fig. 6 is the executed repeatedly in the computer 20.

At step 120, the face image is acquired from the image capture device 12, and at step 122 the eye region is extracted from the acquired face image. Then, at step 124, the degree of eye openness is computed based on the extracted eye region image and stored in a memory (not shown in the drawings). Next, at step 126, determination is made as to whether or not a specific duration has elapsed since starting processing. Processing returns to step 120 when the specific duration has not yet elapsed, and processing proceeds to step 128 then the specific duration has elapsed.

Time series data of degree of eye openness detected during the specific duration is stored in the memory by step 120 to step 126.

At step 128, the plural types of closed eye threshold value stored in the threshold value storage section 28 are imported. At step 130, the plural types of closed eye threshold value acquired at step 128 are employed to extract respective plural types of blinking feature amount based on the degree of eye openness time series data stored in the memory. At above step 130, the large value closed eye threshold value is employed, and based on the degree of eye openness time series data a range of continuous closed-eye state less than the closed eye threshold value is extracted, thereby extracting a continuous closed-eye duration. Furthermore, the standard closed eye threshold value is employed to extract changes exceeding this closed eye threshold value, thereby extracting the number of blinks. Furthermore, the small value closed eye threshold value is employed to extract, based on the degree of eye openness time series data, the range of closed-eye state less than this closed eye threshold value, thereby extracting the proportion of closed-eye.

Next, at step 132, threshold value determination is performed for each of the plural types of blinking feature amount extracted at step 130, and at step 134, determination is made as to whether or not all of the blinking feature amounts were determined to be the corresponding threshold value or greater in the threshold value determination of step 132. The drowsiness determination processing routine is ended when at least one of the types of blinking feature amount is determined to be less than the respective threshold value in the threshold value determination at step 132 above. However, determination is made that the driver is in a nodding-off state when all of the blinking feature amounts are determined to be a blinking feature amount corresponding to a nodding-off state due to being the corresponding threshold value or greater at the threshold value determination of step 132. Then, at step 136, a warning message is displayed on the display device 40, prompting the driver to pay attention to their drowsiness, completing the drowsiness determination processing routine.

As explained above, according to the drowsiness determination apparatus according to the first exemplary embodiment, the standard closed eye threshold value is employed on the degree of eye openness to extract the number of blinks, the small value closed eye threshold value is employed thereon to extract the proportion of closed-eye, and the large value closed eye threshold value is employed to extract the continuous closed-eye duration. Accordingly, plural types of blinking feature amount can be extracted with good precision using the closed eye threshold values appropriate to the respective blinking feature amount, type, enabling state of drowsiness determination to be performed with good precision.

Furthermore, even if noise components are incorporated in detected degree of eye openness time series data due to wrong detection temporality, lost tracking temporality and the like, extraction threshold values are selected according to the type of blinking feature amount to be extracted. Accordingly, since influence due to fluctuations in degree of eye openness and noise on the blinking feature amount desired for extraction can be reduced, nodding-off determination with good precision is enabled.

Explanation now follows regarding a second exemplary embodiment. Note that since the configuration of the drowsiness determination apparatus according to the second exemplary embodiment is similar to the configuration of the drowsiness determination apparatus according to the first exemplary embodiment, the same reference numerals are appended and further explanation thereof is omitted.

The second exemplary embodiment differs from the first exemplary embodiment in the point that the maximum open-eye duration, grouping of blinking, and variance of open-eye duration are extracted as plural types of blinking feature amount.

A feature amount extraction section 30 of the drowsiness determination apparatus according to the second exemplary embodiment employs plural types of closed eye threshold value to extract plural types of blinking feature amount as explained in the following.

First, when extracting a blinking feature amount relating to length of open-eye state in a given time period by derivation using a value corresponding to the length of a portion of open-eye state in a given time period, the feature amount extraction section 30 employs the small value closed eye threshold value thC as the extraction threshold value in order not to be influenced by noise in the open-eye state, to derives the maximum limit extraction range of open-eye state, and to derive a value corresponding to the length of a portion of open-eye state with good precision. Accordingly, the open-eye state of the closed eye threshold value thC or greater is extracted from the degree of eye openness time series data, a value corresponding to the length of the portion of open-eye state in the given time period is derived with good precision, thereby extracting the blinking feature amount.

Furthermore, when extracting the grouping of blinking as a blinking feature amount indicating the number of times blinking is repeated with an inter-blink spacing interval in a given time period, in order not to count as blinks instances when the eye does not fully open, the feature amount extraction section 30 employs the small value closed eye threshold value thC as the extraction threshold value, extracts the number of times the small value closed eye threshold value thC is exceeded in the degree of eye openness time series data. The feature amount extraction section 30 extracts the number of times blinking is repeated at the inter-blink spacing interval in the given time period with good precision, thereby extracting the grouping of blinking.

Furthermore, when a blinking feature amount relating to length of open-eye state in the given time period by derivation using a value corresponding to the entire length of the open-eye state in a given time period is extract, noise in the open-eye state does not readily have an influence. Furthermore, were the closed eye threshold value thC near to the closed-eye state be employed, then the closed eye threshold value thC would sometimes be exceeded even in states in which the eye is not actually fully open, leading to incorrect interpretation as open-eye state. However, the feature amount extraction section 30 employs the large value closed eye threshold value thA as the extraction threshold value in order to derive the open-eye state more accurately, extracting open-eye state of this closed eye threshold value or greater from the degree of eye openness time series data with good precision, and thereby using a value corresponding to the entire length (time) of open-eye duration in the given time period for the blinking feature amount extraction.

The threshold value determination section 32 determines whether or not the blinking feature amounts correspond to a nodding-off state by performing the threshold value determination on each of the respective extracted plural types of blinking feature amount. For example, determination is made as to whether or not the extracted maximum open-eye duration is the threshold value for maximum open-eye duration or smaller, and determination is made as to whether or not the extracted grouping of blinking is the threshold value for grouping of blinking or greater. Furthermore, determination is made as to whether or not the extracted variance of open-eye duration is the threshold value for variance of open-eye duration or greater.

Since other parts of the configuration and processing according to the second exemplary embodiment are similar to those of the first exemplary embodiment, further explanation is omitted.

Thus, the grouping of blinking is extracted employing the small value closed eye threshold value, the variance of open-eye duration is extracted employing the large value closed eye threshold value, and the maximum open-eye duration is extracted employing the small value closed eye threshold value. Accordingly, since plural types of blinking feature amount can be extracted with good precision employing respective closed eye threshold values appropriate to the type of blinking feature amount, state of drowsiness can be determined with good precision.

Note that while explanation was given in the first exemplary embodiment above of an example in which the continuous closed-eye duration, the number of blinks, and the proportion of closed-eye are extracted as the plural types of blinking feature amount, and explanation was given in the second exemplary embodiment above of an example in which the grouping of blinking, the variance of open-eye duration, and the maximum open-eye duration are extracted as the plural types of blinking feature amount, there is no limitation thereto. Configuration may be made such that for the plural types of blinking feature amount, any desired combination is selected from a blinking feature amount related to grouping of blinking, a blinking feature amount related to a number of blinks, a blinking feature amount relating to length of open-eye state in a given time period by derivation using a value corresponding to the entire length of open-eye state in the given time period, a blinking feature amount relating to length of open-eye state in a given time period by derivation using a value corresponding to the length of a portion of open-eye state in the given time period, a blinking feature amount relating to length of closed-eye state in a given time period by derivation using a value corresponding to the entire length of closed-eye state in the given time period, and a blinking feature amount relating to length of closed-eye state in a given time period by derivation using a value corresponding to the length of a portion of the closed-eye state in the given time period. Configuration may be made such that any two types of blinking feature amount are extracted from the above blinking feature amounts, configuration may be made such that any three types of blinking feature amount are extracted from the above blinking feature amounts, configuration may be made such that any four types of blinking feature amount are extracted from the above blinking feature amounts or configuration may be made such that any five types of blinking feature amount are extracted from the above blinking feature amounts. Furthermore, configuration may be made such that all of the above types of blinking feature amount are extracted. In order to extract the blinking feature amount relating to grouping of blinking, the small value closed eye threshold value may be employed for extracting blinks, as explained in the second exemplary embodiment above. In order to extract the blinking feature amount relating to the number of blinks, configuration may be made such that the number of blinks is counted employing the standard closed-eye threshold value, as explained in the first exemplary embodiment above. In order to extract the blinking feature amount relating to the length of open-eye state in the given time period by derivation using a value corresponding to the length of a portion of open-eye state in the given time period, configuration may be made such that the open-eye state is extracted employing the small value closed eye threshold value, as explained in the second exemplary embodiment above. In order to extract the blinking feature amount related to open-eye state in the given time period by derivation using a value corresponding to the entire length of open-eye state in the given time period, configuration may be made such that the open-eye state is extracted using the large value closed eye threshold value, as explained in the second exemplary embodiment above. In order to extract the blinking feature amount relating to the length of closed-eye state in the given time period by derivation using a value corresponding to the length of a portion of closed-eye state in the given time period, configuration may be made such that the closed-eye state is extracted employing the large value closed eye threshold value, as explained in the first exemplary embodiment above. In order to extract the blinking feature amount related to closed-eye state in the given time period by derivation using a value corresponding to the entire length of closed-eye state in the given time period, configuration may be made such that the open-eye state is extracted using the small value closed eye threshold value, as explained in the first exemplary embodiment above.

Furthermore, while explanation in the above first exemplary embodiment and second exemplary embodiment is of examples in which, by threshold value determination, the driver is determined to be in a nodding-off state when all the plural extracted types of the blinking feature amount are determined to be values corresponding to a nodding-off state, there is no limitation thereto. For example, configuration may be made such that, by threshold value determination, the driver is determined to be in a nodding-off state when determined that half or more of the types of blinking feature amount, from out of all of the plural extracted types of blinking feature amount, are values corresponding to a nodding-off state. Furthermore, configuration may be made such that the driver is determined to be in a nodding-off state when at least one type of blinking feature amounts is determined to be a value corresponding to a nodding-off state.

Furthermore, while explanation is given of a case in which the threshold value calculation processing routine and the drowsiness determination processing routine are separately executed, there is no limitation thereto. Configuration may be made such that, after storing degree of eye openness time series data, determination is made as to whether or not the plural types of closed eye threshold value have already been calculated, and processing is performed for calculating the plural types of closed eye threshold value based on the stored degree of eye openness time series data if the plural types of closed eye threshold value have not yet been calculated. Configuration may be made such that subsequently drowsiness determination is then performed based on the stored degree of eye openness time series data.

Furthermore, configuration may be made such that the plural types of closed eye threshold value are employed to extract the plural types of blinking feature amount from degree of eye openness time series data after noise reduction has been performed using a digital filter or the like after, so as to then perform drowsiness determination.

Furthermore, while explanation has been given of examples in which the large value closed eye threshold value and the small value closed eye threshold value are computed from the frequency distribution of the degree of eye openness, there is no limitation thereto. For example, configuration may be made such that a peak value of noise during the open-eye state is computed from the degree of eye openness time series data, so as to calculate a value less than the computed peak value of noise during the open-eye state as the small value closed eye threshold value. Configuration may also be made such that a peak value of noise during the closed-eye state is computed from the degree of eye openness time series data, so as to calculate a value greater than the computed peak value of noise during the closed-eye state as the large value closed eye threshold value. Accordingly, a closed eye threshold value for extracting the open-eye state without influence from noise in open-eye state, and a closed eye threshold value for extracting closed-eye state without influence from noise in the closed-eye state can be derived.

The program according to the present invention may be provided stored on a storage medium, such as a CD-ROM or the like.

Explanation of the Reference Numerals

10 drowsiness determination apparatus
12 image capture device
20 computer
22 eye region extraction section
24 degree of eye openness detection section
26 threshold value calculation section
28 threshold value storage section
30 feature amount extraction section
32 threshold value determination section
34 drowsiness determination section

**Claims**

1. A drowsiness determination apparatus comprising:

an image capture means (12) for capturing a region including an eye of a determination subject;
an openness detection means (24) for detecting a degree of eye openness based on the image captured by the image capture means;
a feature amount extraction means (30) for, based on the degree of eye openness detected by the openness detection means, extracting a plurality of types of blinking feature amount selected from a group consisting of:

a blinking feature amount descriptive of grouping of blinking, derived by employing a threshold value for degree of eye openness smaller than a standard threshold value,
a blinking feature amount descriptive of a number of blinks, derived by employing the standard threshold value and differing from the blinking feature amount related to the grouping of blinking,

a state of drowsiness determination means (34) for determining the state of drowsiness of the determination subject based on the plurality of types of blinking feature amount extracted by the feature amount extraction means

**characterized in that**

the group further consists of:

a blinking feature amount descriptive of length of open-eye state in a given time period, derived by employing a threshold value greater than the standard threshold value and a value corresponding to the entire length of open-eye state in the given time period,
a blinking feature amount descriptive of length of open-eye state in a given time period, derived by employing a threshold value smaller than the standard threshold value and a value corresponding to the length of a portion of open-eye state in the given time period,
a blinking feature amount descriptive of length of closed-eye state in a given time period, derived by employing a threshold value smaller than the standard threshold value and a value corresponding to the entire length of closed-eye state in the given time period, and
a blinking feature amount descriptive of length of closed-eye state in a given time period, derived by employing a threshold value greater than the standard threshold value and a value corresponding to the length of a portion of the closed-eye state in the given time period.

**2.** The drowsiness determination apparatus of claim 1, further comprising a threshold value computation means for, based on the degree of eye openness detected by the openness detection means, computing the standard threshold value, the threshold value greater than the standard threshold value and the threshold value smaller than the standard threshold value.

**3.** The drowsiness determination apparatus of claim 2, wherein the threshold value computation means computes the standard threshold value, the threshold value greater than the standard threshold value and the threshold value smaller than the standard threshold value based on a distribution of the degree of eye openness obtained from the degree of eye openness detected by the openness detection means.

**4.** The drowsiness determination apparatus of claim 3, wherein the threshold value computation means:

extracts a maximum value with larger degree of eye openness and a maximum value with smaller degree of eye openness from two maximum values in the degree of eye openness distribution, together with a minimum value present between the two maximum values; and

computes the standard threshold value based on the minimum value, computes the threshold value smaller than the standard threshold value based on the minimum value and the maximum value of greater degree of eye openness, and computes the threshold value smaller than the standard threshold value based on the minimum value and the maximum value of larger degree of eye openness.

**5.** A program for causing a computer (20) to function as:

an openness detection means (24) for detecting a degree of eye openness based on an image captured by an image capture means for capturing a region including an eye of a determination subject;

a feature amount extraction means (30) for, based on the degree of eye openness detected by the openness detection means, extracting a plurality of types of blinking feature amount selected from a group consisting of:

a blinking feature amount descriptive of grouping of blinking, derived by employing a threshold value for degree of eye openness smaller than a standard threshold value,

a blinking feature amount descriptive of a number of blinks, derived by employing the standard threshold value and differing from the blinking feature amount related to the grouping of blinking;

and a state of drowsiness determination means (34) for determining the state of drowsiness of the determination subject based on the plurality of types of blinking feature amount extracted by the feature amount extraction means.

**characterized in that**

the group further consists of:

a blinking feature amount descriptive of length of open-eye state in a given time period, derived by employing a threshold value greater than the standard threshold value and a value corresponding to the entire length of open-eye state in the given time period,

a blinking feature amount descriptive of length of open-eye state in a given time period, derived by employing a threshold value smaller than the standard threshold value and a value corresponding to the length of a portion of open-eye state in the given time period,

a blinking feature amount descriptive of length of closed-eye state in a given time period, derived by employing a threshold value smaller than the standard threshold value and a value corresponding to the entire length of closed-eye state in the given time period, and

a blinking feature amount descriptive of length of closed-eye state in a given time period, derived by employing a threshold value greater than the standard threshold value and a value corresponding to the length of a portion of the closed-eye state in the given time period.

**Patentansprüche**

**1.** Schläfrigkeitsbestimmungsvorrichtung mit:

einer Bildaufnahmeeinrichtung (12) zum Aufnehmen eines Gebiets einschließlich eines Auges eines Bestimmungssubjekts;

einer Öffnungserfassungseinrichtung (24) zum Erfassen eines Grads einer Augenöffnung basierend auf dem Bild, das durch die Bildaufnahmeeinrichtung aufgenommen wurde;

einer Merkmalsgrößenextraktionseinrichtung (30), um, basierend auf dem Grad der Augenöffnung, der durch die Öffnungserfassungseinrichtung bestimmt wurde, eine Vielzahl von Typen von Blinzelmerkmalsgrößen zu extrahieren, die aus einer Gruppe ausgewählt sind, die besteht aus:

einer Blinzelmerkmalsgröße, die für eine Gruppe des Blinzelns beschreibend ist, die durch Anwenden eines Schwellenwerts, der kleiner als ein Standardschwellenwert ist, auf den Grad der Augenöffnung abgeleitet wird;

einer Blinzelmerkmalsgröße, die für eine Anzahl von Blinzeln beschreibend ist, die durch Anwenden des Standardsschwellenwerts abgeleitet wird, und die sich von der Blinzelmerkmalsgröße, die auf die Gruppe des Blinzelns bezogen ist, unterscheidet,

einer Schläfrigkeitszustandsbestimmungseinrichtung (34) zum Bestimmen des Schläfrigkeitszustands des Bestimmungssubjekts basierend auf der Vielzahl von Typen von Blinzelmerkmalsgrößen, die durch die Merkmalsgrößenextraktionseinrichtung extrahiert wurden,

**dadurch gekennzeichnet, dass**

die Gruppe ferner besteht aus:

einer Blinzelmerkmalsgröße, die für einen offenen Augenzustand in einer gegebenen Zeitperiode beschreibend ist, die durch Anwenden eines Schwellenwertes, der größer als der Standardschwellenwert ist, und einem Wert, der der ganzen Länge des offenen Augenzustandes in der gegebenen Zeitperiode entspricht, abgeleitet wird,

einer Blinzelmerkmalsgröße, die für eine Länge eines offenen Augenzustands in einer gegebenen Zeitperiode beschreibend ist, die durch Anwenden eines Schwellenwertes, der kleiner als der Standardschwellenwert ist, und eines Wertes, der der Länge eines Teils eines offenen Augenzustands in der gegebenen Zeitperiode entspricht, abgeleitet wird,

einer Blinzelmerkmalsgröße, die für eine Länge eines geschlossenen Augenzustands in einer gegebenen Zeitperiode beschreibend ist, die durch Anwenden eines Schwellenwertes, der kleiner als der Standardschwellenwert ist, und eines Wertes, der der ganzen Länge des geschlossenen Augenzustands in der gegebenen Zeitperiode entspricht, abgeleitet wird, und

einer Blinzelmerkmalsgröße, die für eine Länge eines geschlossenen Augenzustands in einer gegebenen Zeitperiode beschreibend ist, die durch Anwenden eines Schwellenwerts, der größer als der Standardschwellenwert ist, und eines Wertes, der der Länge eines Teils des geschlossenen Augenzustands in der gegebenen Zeitperiode entspricht, abgeleitet wird.

2. Schläfigkeitsbestimmungsvorrichtung nach Anspruch 1, ferner mit einer Schwellenwertberechnungseinrichtung zum Berechnen des Standardschwellenwertes, des Schwellenwertes, der größer als der Standardschwellenwert ist, und des Schwellenwertes, der kleiner als der Standardschwellenwert ist, basierend auf dem Grad einer Augenöffnung, die durch die Öffnungserfassungseinrichtung erfasst wird.

3. Schläfrigkeitsbestimmungsvorrichtung nach Anspruch 2, wobei die Schwellenwertberechnungseinrichtung den Standardschwellenwert, den Schwellenwert, der größer als der Standardschwellenwert ist, und den Schwellenwert, der kleiner als der Standardschwellenwert ist, basierend auf einer Verteilung eines Grads an Augenöffnung berechnet, der aus dem Grad einer Augenöffnung, der durch die Öffnungserfassungseinrichtung erfasst wird, erhalten wird.

4. Schläfrigkeitsbestimmungsvorrichtung nach Anspruch 3, wobei die Schwellenwertberechnungseinrichtung:

einen Maximalwert mit einem größeren Grad an Augenöffnung und einen Maximalwert mit einem kleineren Grad an Augenöffnung aus zwei Maximalwerten des Grads einer Augenöffnungsverteilung zusammen mit einem Minimalwert, der zwischen den zwei Maximalwerten vorhanden ist, extrahiert; und

den Standardschwellenwert basierend auf dem Minimalwert berechnet, den Schwellenwert, der kleiner als der Standardschwellenwert ist, basierend auf dem Minimalwert und dem Maximalwert eines größeren Grads einer Augenöffnung berechnet, und den Schwellenwert, der kleiner als der Standardschwellenwert ist, basierend auf dem Minimalwert und dem Maximalwert eines größeren Grads an Augenöffnung berechnet.

5. Programm, um einen Computer (20) zu veranlassen, zu fungieren als:

einer Öffnungserfassungseinrichtung (24) zum Erfassen eines Grads einer Augenöffnung basierend auf dem Bild, das durch die Bildaufnahmeeinrichtung aufgenommen wurde;

einer Merkmalsgrößenextraktionseinrichtung (30), um, basierend auf dem Grad der Augenöffnung, der durch die Öffnungserfassungseinrichtung bestimmt wird, eine Vielzahl von Typen von Blinzelmerkmalsgrößen zu extrahieren, die aus einer Gruppe ausgewählt sind, die besteht aus:

einer Blinzelmerkmalsgröße, die für eine Gruppe des Blinzelns beschreibend ist, die durch Anwenden eines Schwellenwerts, der kleiner als ein Standardschwellenwert ist, auf den Grad der Augenöffnung abgeleitet wird,
einer Blinzelmerkmalsgröße, die für eine Anzahl von Blinzeln beschreibend ist, die durch Anwenden des Standardsschwellenwerts abgeleitet wird, und die sich von der Blinzelmerkmalsgröße, die auf die Gruppe des Blinzelns bezogen ist, unterscheidet,
einer Schläfrigkeitszustandsbestimmungseinrichtung (34) zum Bestimmen des Schläfrigkeitszustands des Bestimmungssubjekts basierend auf der Vielzahl von Typen von Blinzelmerkmalsgrößen, die durch die Merkmalsgrößenextraktionseinrichtung extrahiert wurden,
**dadurch gekennzeichnet, dass**
die Gruppe ferner besteht aus:

einer Blinzelmerkmalsgröße, die für einen offenen Augenzustand in einer gegebenen Zeitperiode beschreibend ist, der durch Anwenden eines Schwellenwertes, der größer als der Standardschwellenwert ist, und einem Wert, der der ganzen Länge des offenen Augenzustandes in der gegebenen Zeitperiode entspricht, abgeleitet wird,
einer Blinzelmerkmalsgröße, die für eine Länge eines offenen Augenzustands in einer gegebenen Zeitperiode beschreibend ist, die durch Anwenden eines Schwellenwertes, der kleiner als der Standardschwellenwert ist, und eines Wertes, der der Länge eines Teils eines offenen Augenzustands in der gegebenen Zeitperiode entspricht, abgeleitet wird,
einer Blinzelmerkmalsgröße, die für eine Länge eines geschlossenen Augenzustands in einer gegebenen Zeitperiode beschreibend ist, die durch Anwenden eines Schwellenwertes, der kleiner als der Standardschwellenwert ist, und eines Wertes, der der ganzen Länge des geschlossenen Augenzustands in der gegebenen Zeitperiode entspricht, abgeleitet wird, und
einer Blinzelmerkmalsgröße, die für eine Länge eines geschlossenen Augenzustands in einer gegebenen Zeitperiode beschreibend ist, die durch Anwenden eines Schwellenwerts, der größer als der Standardschwellenwert ist, und eines Wertes, der der Länge eines Teils des geschlossenen Augenzustands in der gegebenen Zeitperiode entspricht, abgeleitet wird.

**Revendications**

1. Appareil de détermination de somnolence comprenant :

un moyen de capture d'image (12) destiné à capturer une région incluant un œil d'un sujet soumis à détermination ;
un moyen de détection d'ouverture (24) destiné à détecter un degré d'ouverture d'œil sur la base de l'image capturée par le moyen de capture d'image ;
un moyen d'extraction de grandeurs caractéristiques (30) destiné à extraire, sur la base du degré d'ouverture d'œil détecté par le moyen de détection d'ouverture, une pluralité de types de grandeurs caractéristiques de clignement, choisis dans un groupe composé de :

une grandeur caractéristique de clignement représentative d'un groupement de clignements, déterminée par emploi d'une valeur de seuil de degré d'ouverture d'œil inférieure à une valeur de seuil normale,
une grandeur caractéristique de clignement représentative d'un nombre de clignements, déterminée par emploi de la valeur de seuil normale et différant de la grandeur caractéristique de clignement se rapportant au groupement de clignements,

un moyen de détermination d'état de somnolence (34) destiné à déterminer l'état de somnolence du sujet soumis à détermination, sur la base de la pluralité de types de grandeurs caractéristiques de clignement extraits par le moyen d'extraction de grandeurs caractéristiques,
**caractérisé en ce que** :
le groupe se compose en outre de :

une grandeur caractéristique de clignement représentative d'une durée de l'état d'œil ouvert sur une période de temps donnée, déterminée par emploi d'une valeur de seuil supérieure à la valeur de seuil normale et d'une valeur correspondant à la durée totale de l'état d'œil ouvert sur la période de temps donnée,
une grandeur caractéristique de clignement représentative d'une durée de l'état d'œil ouvert sur une période de temps donnée, déterminée par emploi d'une valeur de seuil inférieure à la valeur de seuil normale et d'une valeur correspondant à la durée d'une partie de l'état d'œil ouvert sur la période de temps donnée,
une grandeur caractéristique de clignement représentative d'une durée de l'état d'œil fermé sur une période de temps donnée, déterminée par emploi d'une valeur de seuil inférieure à la valeur de seuil normale et d'une valeur correspondant à la durée totale de l'état d'œil fermé sur la période de temps donnée, et
une grandeur caractéristique de clignement représentative d'une durée de l'état d'œil fermé sur une période de temps donnée, déterminée par emploi d'une valeur de seuil supérieure à la valeur de seuil normale et d'une valeur correspondant à la durée d'une partie de l'état d'œil fermé sur la période de temps donnée.

2. Appareil de détermination de somnolence selon la revendication 1, comprenant en outre un moyen de calcul de valeurs de seuil, destiné à calculer, sur la base du degré d'ouverture d'œil détecté par le moyen de détection d'ouverture, la valeur de seuil normale, la valeur de seuil supérieure à la valeur de seuil normale et la valeur de seuil inférieure à la valeur de seuil normale.

3. Appareil de détermination de somnolence selon la revendication 2, dans lequel le moyen de calcul de valeurs de seuil calcule la valeur de seuil normale, la valeur de seuil supérieure à la valeur de seuil normale et la valeur de seuil inférieure à la valeur de seuil normale, sur la base d'une distribution du degré d'ouverture d'œil obtenue à partir du degré d'ouverture d'œil détecté par le moyen de détection d'ouverture.

4. Appareil de détermination de somnolence selon la revendication 3, dans lequel le moyen de calcul de valeurs de seuil :

extrait une valeur maximale avec degré supérieur d'ouverture d'œil et une valeur maximale avec degré inférieur d'ouverture d'œil à partir de deux valeurs maximales dans la distribution de degré d'ouverture d'œil, conjointement avec une valeur minimale présente entre les deux valeurs maximales ; et
calcule la valeur de seuil normale sur la base de la valeur minimale, calcule la valeur de seuil inférieure à la valeur de seuil normale sur la base de la valeur minimale et de la valeur maximale de degré supérieur d'ouverture d'œil, et calcule la valeur de seuil inférieure à la valeur de seuil normale sur la base de la valeur minimale et de la valeur maximale de degré supérieur d'ouverture d'œil.

5. Programme destiné à faire fonctionner un ordinateur (20) en tant que :

moyen de détection d'ouverture (24) destiné à détecter un degré d'ouverture d'œil sur la base d'une image capturée par un moyen de capture d'image destiné à capturer une région incluant un œil d'un sujet soumis à détermination ;
moyen d'extraction de grandeurs caractéristiques (30) destiné à extraire, sur la base du degré d'ouverture d'œil détecté par le moyen de détection d'ouverture, une pluralité de types de grandeurs caractéristiques de clignement, choisis dans un groupe composé de :

une grandeur caractéristique de clignement représentative d'un groupement de clignements, déterminée par emploi d'une valeur de seuil de degré d'ouverture d'œil inférieure à une valeur de seuil normale,
une grandeur caractéristique de clignement représentative d'un nombre de clignements, déterminée par emploi de la valeur de seuil normale et différant de la grandeur caractéristique de clignement se rapportant au groupement de clignements ;

et moyen de détermination d'état de somnolence (34) destiné à déterminer l'état de somnolence du sujet soumis à détermination, sur la base de la pluralité de types de grandeurs caractéristiques de clignement, extraits par le moyen d'extraction de grandeurs caractéristiques,
**caractérisé en ce que** :
le groupe se compose en outre de :

une grandeur caractéristique de clignement représentative d'une durée de l'état d'œil ouvert sur une période de temps donnée, déterminée par emploi d'une valeur de seuil supérieure à la valeur de seuil normale et d'une valeur correspondant à la durée totale de l'état d'œil ouvert sur la période de temps donnée,
une grandeur caractéristique de clignement représentative d'une durée de l'état d'œil ouvert sur une période de temps donnée, déterminée par emploi d'une valeur de seuil inférieure à la valeur de seuil normale et d'une valeur correspondant à la durée d'une partie de l'état d'œil ouvert sur la période de temps donnée,
une grandeur caractéristique de clignement représentative d'une durée de l'état d'œil fermé sur une période de temps donnée, déterminée par emploi d'une valeur de seuil inférieure à la valeur de seuil normale et d'une valeur correspondant à la durée totale de l'état d'œil fermé sur la période de temps donnée, et
une grandeur caractéristique de clignement représentative d'une durée de l'état d'œil fermé sur une période de temps donnée, déterminée par emploi d'une valeur de seuil supérieure à la valeur de seuil normale et d'une valeur correspondant à la durée d'une partie de l'état d'œil fermé sur la période de temps donnée.

EP 2 351 524 B1

FIG.1

10

20

12 — IMAGE CAPTURE DEVICE

22 — EYE REGION EXTRACTION SECTION

24 — DEGREE OF EYE OPENNESS DETECTION SECTION

26 — THRESHOLD VALUE CALCULATION SECTION

28 — THRESHOLD VALUE STORAGE SECTION

30 — FEATURE AMOUNT EXTRACTION SECTION

32 — THRESHOLD VALUE DETERMINATION SECTION

34 — DROWSINESS DETERMINATION SECTION

36 — DISPLAY CONTROLLER

40 — DISPLAY DEVICE

# FIG.2

IDEAL DEGREE OF EYELID OPENNESS WAVEFORM

DEGREE OF EYE OPENNESS WAVEFORM OBTAINED BY IMAGE PROCESSING

OPEN-EYE STATE

CONTINUOUS CLOSED-EYE DURATION OBTAINED FROM EXTRACTION THRESHOLD VALUE

EXTRACTION THRESHOLD VALUE

CONTINUOUS CLOSED-EYE DURATION OBTAINED FROM CLOSED EYE THRESHOLD VALUE

CLOSED EYE THRESHOLD VALUE

CONTINUOUS CLOSED-EYE DURATION THAT EMPLOYED CLOSED EYE THRESHOLD VALUE IS READILY INFLUENCED BY NOISE IN CLOSED-EYE TIME

ACTUAL CONTINUOUS CLOSED-EYE DURATION

DEGREE OF EYE OPENNESS

CLOSED-EYE STATE

TIME

EP 2 351 524 B1

FIG.4

## FIG.5

```
( THRESHOLD VALUE CALCULATION PROCESSING ROUTINE )
                            │
                            ▼
        ┌─────────────────────────────────┐
        │       ACQUIRE FACE IMAGE         │~100
        └─────────────────────────────────┘
                            │
        ┌─────────────────────────────────┐
        │        EXTRACT EYE REGION        │~102
        └─────────────────────────────────┘
                            │
        ┌─────────────────────────────────┐
        │  DETECT AND STORE DEGREE OF EYE  │~104
        │            OPENNESS              │
        └─────────────────────────────────┘
                            │       106
                      ◇─────────────◇
                 ╱    HAS             ╲        N
                ◇ A SPECIFIC DURATION  ◇────────────┐
                 ╲     ELAPSED?       ╱             │
                      ◇─────────────◇
                            │ Y
        ┌─────────────────────────────────┐
        │ COMPUTE FREQUENCY DISTRIBUTION OF│~108
        │    DEGREE OF EYE OPENNESS        │
        └─────────────────────────────────┘
                            │
        ┌─────────────────────────────────┐
        │ COMPUTE EACH TYPE OF THRESHOLD VALUE │~110
        └─────────────────────────────────┘
                            │
        ┌─────────────────────────────────┐
        │ STORE EACH TYPE OF THRESHOLD VALUE │~112
        └─────────────────────────────────┘
                            │
                      (    END    )
```

# FIG.6

```
DROWSINESS DETERMINATION
PROCESSING ROUTINE

ACQUIRE FACE IMAGE              ~120

EXTRACT EYE REGION             ~122

DETECT AND STORE DEGREE        ~124
OF EYE OPENNESS

            126
          HAS
   SPECIFIC DURATION              N
      ELAPSED?

            Y

IMPORT EACH TYPE OF THRESHOLD VALUE    ~128

EXTRACT EACH TYPE OF BLINKING FEATURE  ~130
AMOUNT

PERFORM THRESHOLD VALUE DETERMINATION FOR  ~132
EACH TYPE OF BLINKING FEATURE AMOUNT

                134
          ARE ALL
N    THEIR RESPECTIVE
   THRESHOLD VALUES OR
        GREATER?

            Y

DISPLAY WARNING               ~136

            END
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004041485 A
- JP H03286887B2 B
- JP 3286887 B
- US 5786765 A